# EUROPEAN PATENT APPLICATION

(11) **EP 4 356 904 A1**
(43) Date of publication of application: **24.04.2024**
(21) Application number: 23204467.7
(22) Date of filing: 18.10.2023
(51) Int. Cl.: A61K 9/20, A61K 9/28, A61K 31/4439

(54) **A CAPSULE COMPRISING LANSOPRAZOLE**

(30) Priority: 19.10.2022 TR 202215878
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR); SARP, Onder, Istanbul (TR)
(74) Representative: Sevinç, Erkan

(57) **Abstract**

The present invention relates to pharmaceutical composition in the form of capsule having enteric coating microtablets comprising lansoprazole wherein microtablets comprising maize starch as a diluent and one more diluent. The capsule is obtained by a process which is simple, rapid, cost effective, time-saving and industrially convenient process.

## Description

### Field of the Invention

The present invention relates to pharmaceutical composition in the form of capsule having enteric coating microtablets comprising lansoprazole wherein microtablets comprising maize starch as a diluent and one more diluent. The capsule is obtained by a process which is simple, rapid, cost effective, time-saving and industrially convenient process.

### Background of the Invention

Lansoprazole, chemical name 2-(((3-methyl-4-(2,2,2-trifluoroethoxy)-2-pyridyl)methyl) sulfinyl)-1H-benzoyl Imidazole, brownish white crystalline powder, easily soluble in dimethylamide, soluble in methanol, insoluble in ethanol and ether, almost insoluble in water. It has a chemical structure which is shown in the Formula I.

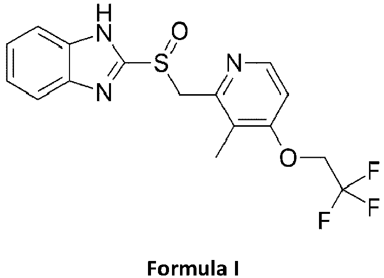

Lansoprazole is the second proton pump inhibitor anti-ulcer drug developed by Takeda Corporation of Japan after omeprazole.1992ln 1999, Japan's Takeda Pharmaceutical Co., Ltd. and Houde Company first officially put it on the market in France, and in May 1995, it was approved by the FDA to be listed in the United States.

Lansoprazole is extremely unstable under acidic conditions, and is degraded to inactive components in a short time under low pH environment, so it is easily degraded in gastric acid environment. In order to improve the bioavailability of the drug, it needs to be prepared as an enteric-coated preparation.

Chinese Patent Application No.: 201010245689.6 discloses a lansoprazole enteric capsule formulation and preparation method, using sodium bicarbonate as a stabilizer, cross-linked polyvinylpyrrolidone as a disintegrant, and acrylic resin as an enteric coating agent.

U.S. Patent No. 6,328,994 discloses an orally disintegrable lansoprazole tablet which comprises fine enteric coated granules having an average particle diameter of 400µπ 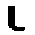 . or less.

Therefore, it is still necessary to prepare the lansoprazole enteric-coated capsule that a kind of related substance content is low, the desired stability and is suitable for industrialized production.

### Detailed Description of the Invention

The main object of the present invention is to eliminate problems caused by active substance and bringing additional advantages to the relevant prior art. To explain in more detail, the main purpose is to provide a pharmaceutical composition in the form of capsule having enteric coating microtablets having high physical and chemical stability and a long shelf life by the help of selection of excipients.

Another object of the present invention is to obtain a pharmaceutical composition in the form of capsule having enteric coating microtablets providing improved dissolution profile, it also is cost effective.

Another object of the present invention is to obtain a pharmaceutical composition in the form of capsule having enteric coating microtablets providing improved excellent pharmacomechanic properties, such as flowability, compressibility and content uniformity.

In particular, there is a need to combine an acid-labile physiologically active substance, with basic inorganic salts and so forth for stability, and further to coat with coating layers such as an enteric layer. It is an important problem to produce a small enteric coated microtablets, even though it contains the acid-labile physiologically active substance in small content. In this invention, a pharmaceutical composition in the form of capsule having enteric coating microtablets is used. The composition provides the desired stability and the desired profile. Also, microtablets have content uniformity and flowability.

The microtablets are cylindrical with a flat or convex upper side and lower side and with a diameter and height which are preferably approximately equal and, independently of one another, preferably have a tablet size of from about 1 mm to about 5 mm, more preferably from about 1.5 mm to about 4.5 mm. The microtablets have a tablet weight of about 1 mg to about 50 mg. Preferably, the microtablets have a tablet weight of about 2 mg to about 35 mg.

According to one embodiment of the present invention, a pharmaceutical composition in the form of capsule having enteric coating microtablets comprising lansoprazole wherein; microtablets comprising maize starch as a diluent and one more diluent. In our formulation, maize starch also shows the binding feature and helps to provide the desired dissolution profile.

According to one embodiment of the present invention, the amount of lansoprazole is between 3.0 % and 20.0% by weight in the total composition. Preferably, the amount of lansoprazole is between 5.0 % and 13.0% by weight in the total composition.

Suitable diluents are selected from the group comprising microcrystalline cellulose, mannitol, lactose monohydrate, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment of the present invention, the diluent is microcrystalline cellulose or lactose or mixtures thereof.

According to one embodiment of the present invention, the diluent is microcrystalline cellulose.

According to one embodiment of the present invention, the diluent is lactose.

According to one embodiment of the present invention, the amount of diluents is between 30.0 % and 75.0% by weight in the total composition. Preferably, the amount of diluents is between 40.0 % and 60.0% by weight in the total composition. The amount provides the desired content uniformity. Since the amount of active ingredient is low compared to the whole composition, it is important to ensure content uniformity.

According to one embodiment of the present invention, the microtablets comprise further at least one pharmaceutically acceptable excipient which is selected from the group comprising disintegrants, binders, surfactants, ph modifiers, lubricants, glidants or mixtures thereof.

Suitable disintegrants are selected from the group comprising croscarmellose sodium, crospovidone, alginic acid, alginates, ion-exchange resins, magnesium aluminium silica, sodium carboxymethyl cellulose, calcium silicate, sodium starch glycolate, carboxymethyl cellulose, calcium carboxymethyl cellulose, docusate sodium, guar gum, low substituted hydroxypropyl cellulose, polyacryline potassium, poloxamer, povidone, sodium alginate, sodium glycine carbonate, or mixtures thereof.

According to one embodiment of the present invention, the disintegrant is croscarmellose sodium.

According to one embodiment of the present invention, the amount of disintegrant is between 1.0 % and 8.0% by weight in the total composition.

Suitable binders are selected from the group comprising hydroxypropyl cellulose, polyvinylpyrrolidone, natural gums, gelatin, collagen, agar, alginates, co-povidone, sodium alginate, gum, guar gum, starch mucilage, acacia mucilage, bentonite, laponit, cetostearyl alcohol, pullulan, polydextrose, polyethylene oxide, pectin, aluminia hydroxide, hyaluronic acid or mixtures thereof.

According to one embodiment of the present invention, the binder is hydroxypropyl cellulose.

According to one embodiment of the present invention, the amount of binder is between 0.1 % and 5.0% by weight in the total composition.

Suitable surfactants are selected from the group comprising polyoxyethylene (20) sorbitan monooleate (polysorbate 80), sodium dodecyl sulphate, sodium lauryl sulfate, cetylpyridinium chloride, docusate sodium, lauric acid, phospholipids, cetrimide or mixtures thereof.

According to one embodiment of the present invention, the surfactant is polyoxyethylene (20) sorbitan monooleate. In the present invention, it provides the desired profile. It also helps to provide flowability.

According to one embodiment of the present invention, the amount of surfactant is between 0.1 % and 3.0% by weight in the total composition.

An enteric coating protects the lansoprazole in microtablet from the acidic environment of the stomach, permitting it to be released in the higher pH environment of the lower GI tract. So, enteric coating provides to release the active ingredient gradually and predictably over a 12-hour to 24-hour period.

Suitable pH modifiers are selected from the group comprising magnesium carbonate, aluminum potassium sulfate, anhydrous citric acid, anhydrous disodium hydrogen phosphate, potassium carbonate, anhydrous sodium dihydrogen phosphate, dibasic potassium sulfate, calcium carbonate, dilute hydrochloric acid, monobasic potassium phosphate, phosphoric acid, adipic acid, sodium acetate, sodium bicarbonate, sodium carbonate, sodium citrate, sodium dihydrogen phosphate dihydrate, tribasic sodium phosphate or mixtures thereof.

According to one embodiment of the present invention, the pH modifier is magnesium carbonate.

According to one embodiment of the present invention, the amount of pH modifier is between 1.0 % and 8.0% by weight in the total composition.

Suitable glidants are selected from the group comprising talc, colloidal silica anhydrous, colloidal silicon dioxide, calcium silicate, aluminium silicate, magnesium silicate, magnesium oxide, starch or mixtures thereof.

According to one embodiment of the present invention, the glidant is talc or colloidal silica anhydrous or mixtures thereof. Using these glidants provides the flowability.

According to one embodiment of the present invention, the amount of glidant is between 0.1 % and 9.0% by weight in the total composition.

Suitable lubricants are selected from the group comprising magnesium stearate, silica, calcium stearate, stearic acid, polyethylene glycol, sodium stearyl fumarate, sodium lauryl sulfate, magnesium lauryl sulfate, fumaric acid, glyceryl palmito sulfate, hydrogenated vegetable oil, zinc stearate or mixtures thereof.

According to one embodiment of the present invention, the lubricant is magnesium stearate.

According to one embodiment of the present invention, the amount of lubricant is between 0.4 % and 3.0% by weight in the total composition.

According to one embodiment of the present invention, microtablets comprises;
- Lansoprazole
- Magnesium carbonate
- Maise starch
- Lactose or MCC
- HPC

The methacrylic acid copolymer is used alone or in combination with other another enteric polymers to achieve the desired delayed release profile. It also helps to provide stability.

According to one embodiment of the present invention, the enteric coating comprises;
- Metacrylic acid copolymer dispersion,
- Sodium hydroxide,
- Polysorbate 80.

According to one embodiment of the present invention, the capsule is obtained by using a wet granulation method therefore, a simple and low-cost production method was employed. Wet granulation process efficiently counteracts segregation, so it can achieve good dissolution and disintegration properties. Solvents are used in the process.

The solvent is selected from the group comprising water, acetone, ethanol, isopropanol or mixtures thereof. Preferably, ethanol is used in the present invention. Especially, using ethanol prevents the degradation of lansoprazole during manufacturing process. So, it helps to provide the desired stability.

Physicochemical properties are important parameters in tablet formulation. It can be a problem especially in formulations containing a low amount of active substance. In a formulation, a problem of physicochemical properties with these negatively affects the dissolution profile and stability. We have found in this invention that wet granulation with a solvent comprising ethanol overcomes these problems.

Lansoprazole is used small proportion that can lead to considerable problems during the manufacture of the composition with regard to the uniformity of the content of active agent in the individual composition units. Because of problems uniformity of the content, the active substance may interact with several excipients. It reflects that content uniformity play important role in the dissolution of the drug. Especially for this reason, we used a binder and a surfactant in the preparation of granulation solution comprising ethanol. We saw that the use of a binder and a surfactant in the preparation of granulation solution surprisingly achieved the desired stability, content uniformity, flowability and homogenity.

According to one embodiment of the present invention, a process for the preparation of a capsule comprises lansoprazole the following steps;
a) Mixing Lansoprazole, maize starch and a diluent, pH modifier and a half of a disintegrant,
b) Granulating the dry mixtures with a binder and a surfactant in a solution comprising ethanol.

According to one embodiment of the present invention, a process for the preparation of a capsule comprises lansoprazole the following steps;
a) Mixing Lansoprazole, maize starch and a diluent, magnesium carbonate and a half of croscarmellose sodium,
b) Granulating the dry mixtures with HPC and a polysorbate 80 in a solution comprising ethanol.

### Example 1: Microtablets are obtained by wet granulation

| **Ingredients** | | **Amount (% by weight of the total formulation)** |
|---|---|---|
| Lansoprazole | | 3.0 - 20.0 |
| Magnesium carbonate | | 1.0 - 8.0 |
| Lactose or MCC | | 45.0 - 70.0 |
| Maize starch | | 1.0 - 8.0 |
| HPC | | 0.1 - 5.0 |
| Polysorbate 80 | | 0.1 - 3.0 |
| Croscarmellose sodium | | 1.0 - 8.0 |
| Talc | | 0.1 - 4.5 |
| Colloidal silica anhydrous | | 0.1 - 4.5 |
| Magnesium stearate | | 0.4 - 3.0 |

| | **Microtablet** | |
|---|---|---|
| Enteric coating | | 10.0 - 30.0 |
| **Total Enteric coating microtablet** | | **100** |

### Example 2: Microtablets are obtained by wet granulation

| **Ingredients** | | **Amount (% by weight of the total formulation)** |
|---|---|---|
| Lansoprazole | | 8.4 |
| Magnesium carbonate | | 3.1 |
| Lactose or MCC | | 46.7 |
| Maize starch | | 6.2 |
| HPC | | 0.8 |
| Polysorbate 80 | | 0.8 |
| Croscarmellose sodium | | 3.1 |
| Talc | | 3.8 |
| Colloidal silica anhydrous | | 3.8 |
| Magnesium stearate | | 1.5 |
| | **Microtablet** | |
| Enteric coating | | 21.8 |
| **Total Enteric coating microtablet** | | **100** |

A process for example 1 or 2;
a) Mixing Lansoprazole, Maize starch, Magnesium carbonate, Lactose or MCC, a half of croscarmellose sodium for 15 minutes,
b) Granulating the dry mixtures with HPC and Polysorbate 80 in a solution comprising ethanol,
c) Sieving the wet granules into 2.0 mm,
d) Drying the granules and sieving the dry granules into 0.85 mm,
e) Adding the remaining part of croscarmellose sodium, talc, colloidal silica anhydrous and then mixing for 5 minutes,
f) Adding 0.6 mm sieved Magnesium stearate and then mixing for 3 minutes,
g) Obtaining microtablets,
h) Coating the microtablets with enteric coating having Metacrylic acid copolymer dispersion,
i) Filling the microtablets into capsules.

### Enteric coating

| **Ingredients** |
|---|
| Metacrylic acid copolymer dispersion |
| Sodium hydroxide |
| Polysorbate 80 |
| Propilen glikol |
| Titanium dioxide |
| Cetyl alcohol |

## Claims

1. A pharmaceutical composition in the form of capsule having enteric coating microtablets comprising lansoprazole wherein; microtablets comprising maize starch as a diluent and one more diluent.

2. The pharmaceutical composition according to claim 1, wherein diluents are selected from the group comprising microcrystalline cellulose, mannitol, lactose monohydrate, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

3. The pharmaceutical composition according to claim 1, wherein the diluent is microcrystalline cellulose or lactose or mixtures thereof.

4. The pharmaceutical composition according to claim 1, wherein the amount of diluents is between 30.0 % and 75.0% by weight in the total composition.

5. The pharmaceutical composition according to claim 1, wherein the microtablets comprise further at least one pharmaceutically acceptable excipient which is selected from the group comprising disintegrants, binders, surfactants, ph modifiers, lubricants, glidants or mixtures thereof.

6. The pharmaceutical composition according to claim 5, wherein binders are selected from the group comprising hydroxypropylcellulose, polyvinylpyrrolidone, natural gums, gelatin, collagen, agar, alginates, co-povidone, sodium alginate, gum, guar gum, starch mucilage, acacia mucilage, bentonite, laponit, cetostearyl alcohol, pullulan, polydextrose, polyethylene oxide, pectin, aluminia hydroxide, hyaluronic acid or mixtures thereof.

7. The pharmaceutical composition according to claim 5, wherein surfactants are selected from the group comprising polyoxyethylene (20) sorbitan monooleate (polysorbate 80), sodium dodecyl sulphate, sodium lauryl sulfate, cetylpyridinium chloride, docusate sodium, lauric acid, phospholipids, cetrimide or mixtures thereof.

8. The pharmaceutical composition according to claim 7, wherein the surfactant is polyoxyethylene (20) sorbitan monooleate.

9. The pharmaceutical composition according to claim 5, wherein pH modifiers are selected from the group comprising magnesium carbonate, aluminum potassium sulfate, anhydrous citric acid, anhydrous disodium hydrogen phosphate, potassium carbonate, anhydrous sodium dihydrogen phosphate, dibasic potassium sulfate, calcium carbonate, dilute hydrochloric acid, monobasic potassium phosphate, phosphoric acid, adipic acid, sodium acetate, sodium bicarbonate, sodium carbonate, sodium citrate, sodium dihydrogen phosphate dihydrate, tribasic sodium phosphate or mixtures thereof.

10. The pharmaceutical composition according to claim 5, wherein microtablets comprises;
• Lansoprazole
• Magnesium carbonate
• Maise starch
• Lactose or MCC
• HPC

11. The pharmaceutical composition according to claim 1, wherein the enteric coating comprises;
- Metacrylic acid copolymer dispersion,
- Sodium hydroxide,
- Polysorbate 80.

12. The pharmaceutical composition according to claim 1, wherein the capsule is obtained by using a wet granulation method.

13. A process for the preparation of a capsule comprises lansoprazole the following steps;
a) Mixing Lansoprazole, maize starch and a diluent, ph modifier and a half of a disintegrant,
b) Granulating the dry mixtures with a binder and a surfactant in a solution comprising ethanol.
